# EUROPÄISCHE PATENTANMELDUNG

(11) **EP 2 311 933 A1**
(43) Veröffentlichungstag der Anmeldung: **20.04.2011**
(21) Anmeldenummer: 11001063.4
(22) Anmeldetag: 24.06.2009
(51) Int. Cl.: C12M 1/107

(54) **Erdbeckenfermenter**

(30) Priorität: 25.06.2008 AT 10142008
(62) Teilanmeldung aus: 09768604.2
(71) Anmelder: Sattler AG, 8041 Graz (AT)
(72) Erfinder: Die Erfindernennung liegt noch nicht vor
(74) Vertreter: Ellmeyer, Wolfgang

(57) **Zusammenfassung**

Erdbeckenfermenter (1) mit einem abgesenkten Beckenboden (3), der mit einer Dichtungsschicht (4) abgedeckt ist und ein Erdbecken (19) ausbildet, wobei zumindest eine gasdichte Abdeckung (7, 8) vorgesehen ist, und wobei Zu- und Ableitungen (14, 15, 16, 67, 68, 69) und zumindest ein Rührwerk (9) vorgesehen sind, wobei die gasdichte Abdeckung (7, 8) durch eine gasdichte Klemmung auf der Beckenkrone (2) befestigt ist.

## Beschreibung

Die Erfindung betrifft einen Erdbeckenfermenter mit einem abgesenkten Beckenboden, der mit einer Dichtungsschicht abgedeckt ist und ein Erdbecken ausbildet, wobei zumindest eine gasdichte Abdeckung vorgesehen ist, und wobei Zu-und Ableitungen und zumindest ein Rührwerk vorgesehen sind.

Erdbeckenfermenter zur Biogasproduktion dienen zumeist dem Vergären von Gülle oder hochbelasteten Abwässern und erlauben aufgrund ihrer Bauweise eine sehr kostengünstige Errichtung und ein einfaches Betreiben.

Die meisten Erdbeckenfermenter sind im Wesentlichen aus Erdbecken gebildet, die ein- oder zweischalig mit Folien ausgekleidet und mit einer gasdichten, durch den Gasdruck gestützten Abdeckung oben abgeschlossen sind, wobei die Stabilität der Abdeckung durch Vorsehen eines Ballasts gewährleistet ist. Das Rühren erfolgt, wenn überhaupt, hydraulisch durch Umwälzen des Substrates mit externen Pumpen oder durch das Einblasen von Biogas am Fermenterboden.

Aufgabe der Erfindung ist es, einen Erdbeckenfermenter der eingangs genannten Art anzugeben, der bei einfacher Bauweise einen sicheren und dichten Betrieb ermöglicht.

Erfindungsgemäß wird dies dadurch erreicht, dass der Erdbeckenfermenter eine umlaufende Beckenkrone aufweist, die durch ein Fundament gebildet ist, und dass die gasdichte Abdeckung durch eine gasdichte Klemmung auf der Beckenkrone befestigt ist.

Die Beckenkrone bietet somit eine sichere Verankerung der gasdichten Abdeckung.

Nachfolgend wird die Erfindung anhand der in den Zeichnungen dargestellten Ausführungsbeispiele eingehend erläutert. Es zeigt dabei
Fig.1 einen Schnitt durch eine Ausführungsform des erfindungsgemäßen Erdbeckenfermenters;
Fig.2 einen Schnitt durch eine weitere Ausführungsform des erfindungsgemäßen Erdbeckenfermenters;
Fig.3 einen Schnitt durch eine weitere Ausführungsform des erfindungsgemäßen Erdbeckenfermenters;
Fig.4 einen Schnitt durch ein Detail einer weiteren Ausführungsform des erfindungsgemäßen Erdbeckenfermenters;
Fig.5 einen Schnitt durch ein Detail einer weiteren Ausführungsform des erfindungsgemäßen Erdbeckenfermenters;
Fig.6 eine Draufsicht auf eine weitere Ausführungsform des erfindungsgemäßen Erdbeckenfermenters;
Fig.7 einen Querschnitt quer zur Längsachse des Erdbeckenfermenters gemäß
Fig.6;

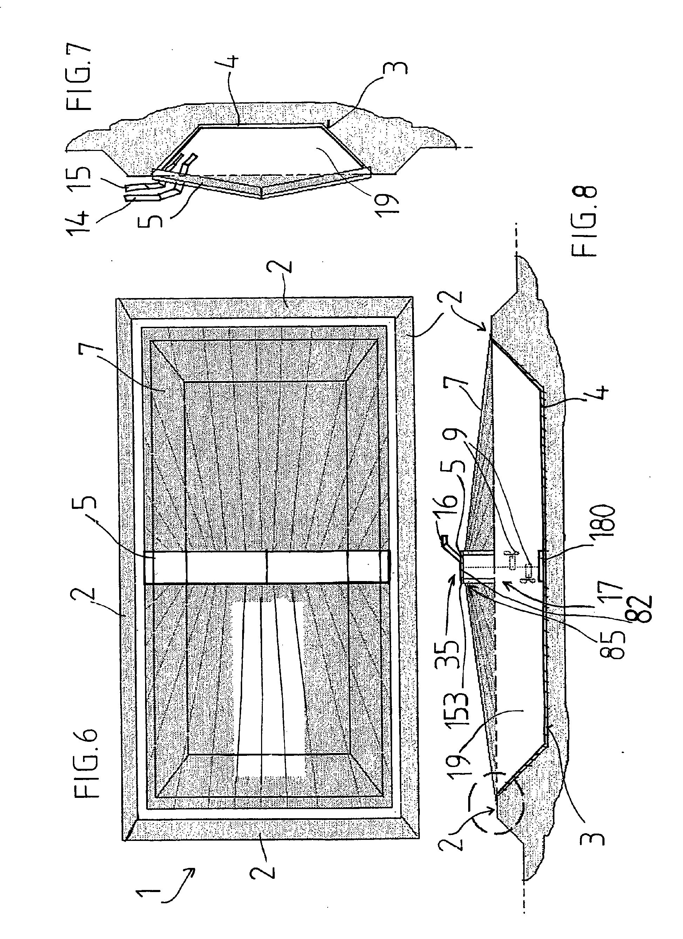

Fig.8 einen Längsquerschnitt des Erdbeckenfermenters gemäß Fig. 6;

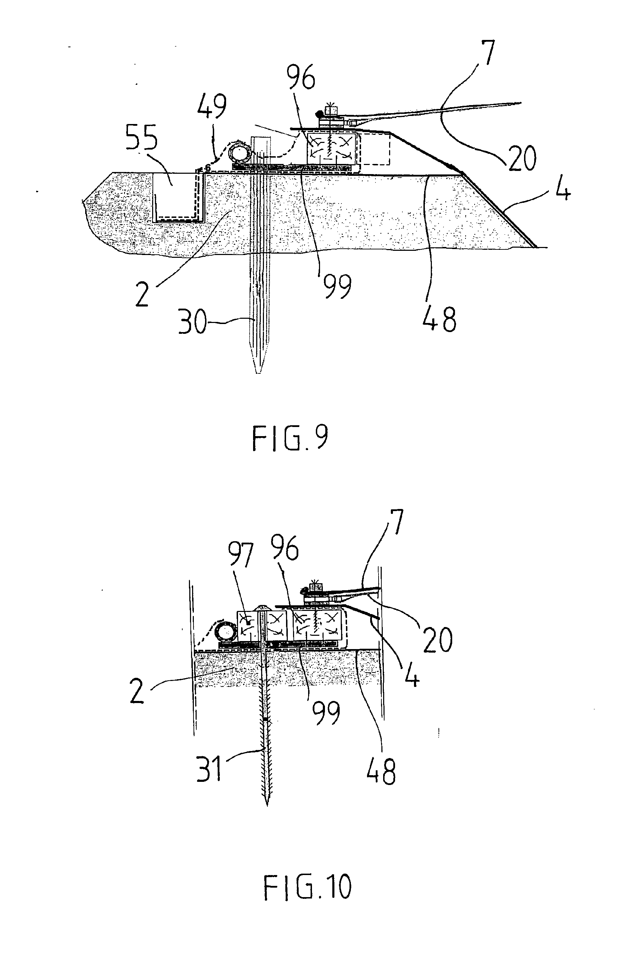

Fig.9 zeigt ein Detail einer Ausführungsvariante zu Fig.6 im Schnitt;
Fig.10 zeigt ein Detail einer weiteren Ausführungsvariante zu Fig.6 im Schnitt;

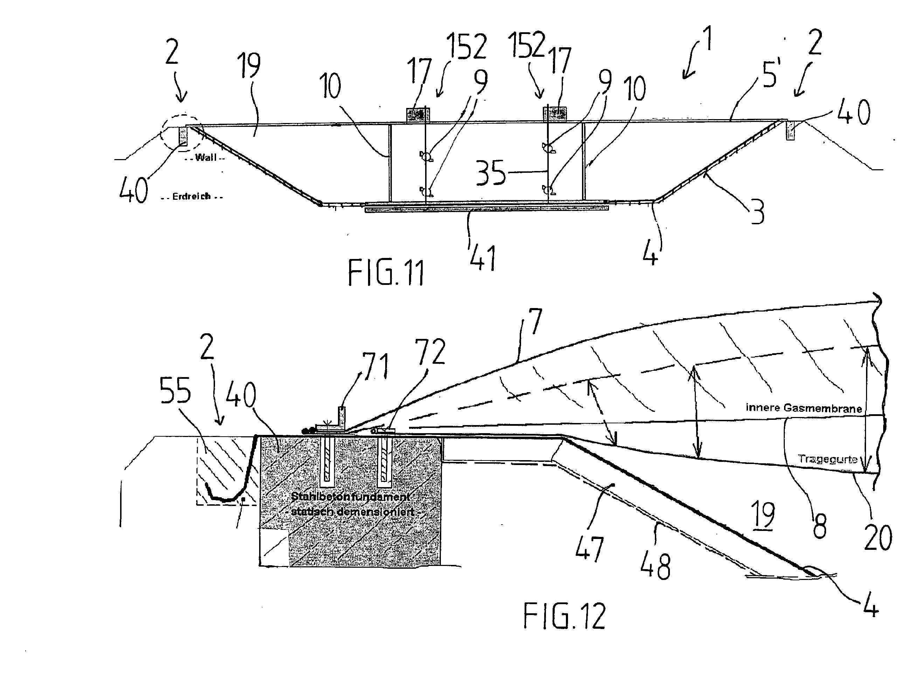

Fig.11 einen Querschnitt quer zur Längsachse eines weiteren Ausführungsbeispiels des erfindungsgemäßen Erdbeckenfermenters;
Fig.12 einen Schnitt durch ein Detail des Erdbeckenfermenters gemäß Fig.11;

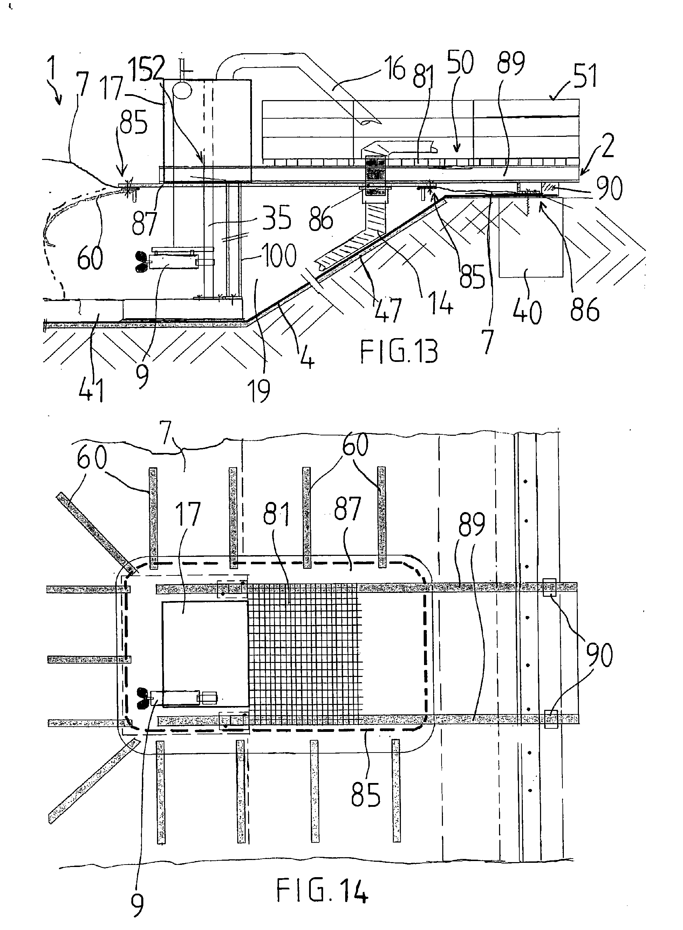

Fig.13 einen teilweisen Schnitt durch eine weitere Ausführungsform des erfindungsgemäßen Erdbeckenfermenters;
Fig.14 eine Draufsicht auf das Detail gemäß Fig.13;

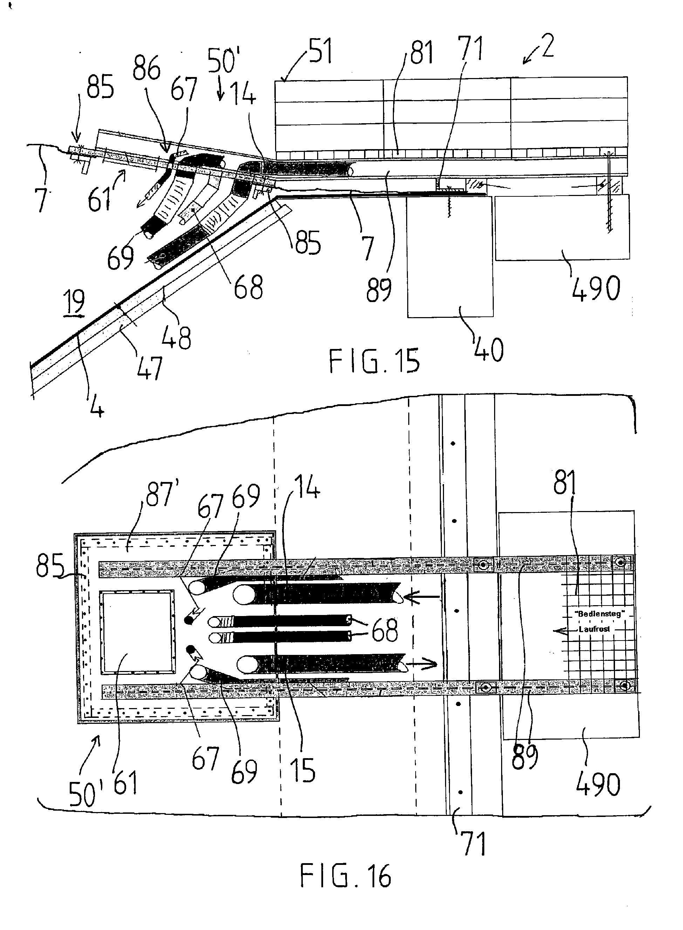

Fig.15 einen teilweisen Schnitt durch eine weitere Ausführungsform des erfindungsgemäßen Erdbeckenfermenters;
Fig.16 eine Draufsicht auf das Detail gemäß Fig.15;

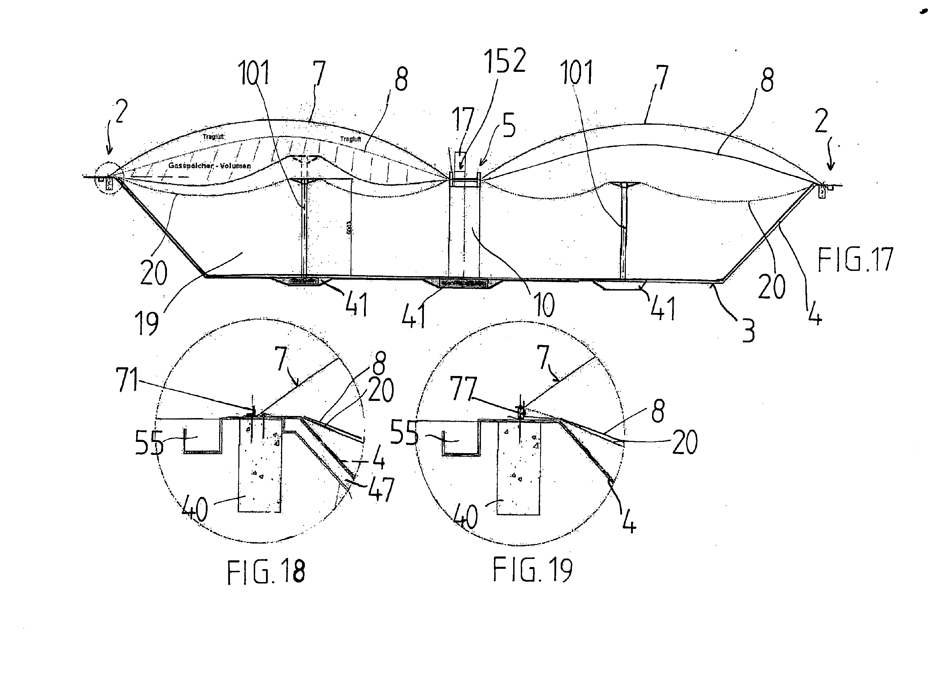

Fig.17 einen Längsschnitt durch eine weitere Ausführungsform des erfindungsgemäßen Erdbeckenfermenters;
Fig.18 einen Schnitt durch eine Variante eines Details des Erdbeckenfermenters gemäß Fig.17 und
Fig.19 einen Schnitt durch eine weitere Variante eines Details des Erdbeckenfermenters gemäß Fig.17.

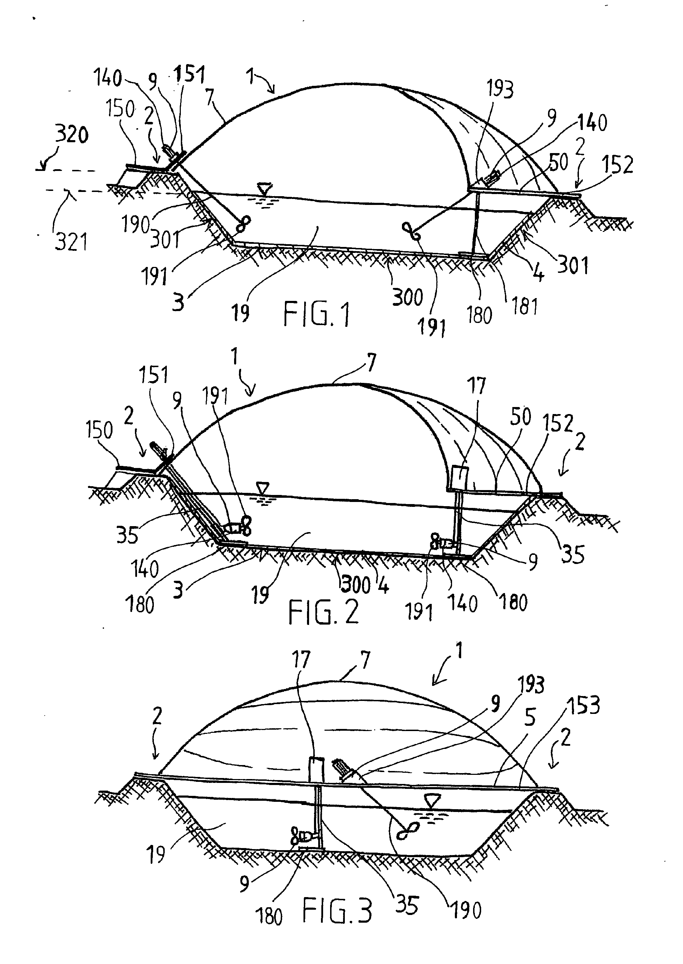

Fig.1 zeigt einen Erdbeckenfermenter 1 mit einem abgesenkten Beckenboden 3, der seitliche Wandschrägen 301 sowie eine tiefliegende Beckensohle 300 aufweist und der mit einer Dichtungsschicht 4 abgedeckt ist und ein Erdbecken 19 ausbildet, an dessen Beckenkrone 2 eine gasdichte Abdeckung, zum größten Teil in Form einer Gasmembran 7, festgelegt ist. Die Gasmembran 7 wird im Betrieb durch den aufgrund der Fermentation sich ausbildenden Innen-Gasdruck in einem mehr oder weniger stark aufgeblähten Zustand vorliegen. Es ist somit in diesem Ausführungsbeispiel die gasdichte Abdeckung ohne zusätzliche Tragekonstruktion sondern nur durch die Gasmembran 7 verwirklicht, die bereichsweise mit ihrem Rand an der Beckenkrone 2 eingespannt ist.

Da das Erdbecken 19 in bestimmten Zeitabständen von außen befüllt und wieder entleert wird, sind Zu- und Ableitungen (nicht dargestellt) vorgesehen, über die Substrat, z.B. Gülle zugeführt bzw. entnommen werden kann. Der Füllstand des Substrats ist in Fig.1 auf dem mit dem Bezugszeichen 321 gezeigten Niveau. Darüber hinaus können weitere Zu- und Ableitungen in das bzw. aus dem Erdbecken 19 führen. Das bei der Fermentation entstehende Gas wird z.B. über eine Gas-Ableitung (nicht dargestellt) abgeleitet, die mit dem Innenraum des Erdbeckenfermenters 1 kommuniziert. Die Dichtungsschicht 4 kann aus einer ein-oder mehrlagigen Kunststofffolie gebildet sein.

Für ausreichende Bewegung des Erdbeckeninhalts wird durch Rührwerke 9 gesorgt, die in allen geeigneten, dem Fachmann bekannten Ausführungsformen vorliegen können.

Erfindungsgemäß ist jeweils eine Auflagerstelle 151, 152 für die Rührwerke 9 im Wesentlichen auf der - in Fig.1 mit dem Bezugszeichen 320 versehenen - Höhe der Beckenkrone 2 und innerhalb des von der Beckenkrone 2 umschlossenen Bereichs vorgesehen. Die Anzahl der Rührwerke 9 und die Art ihrer Ausführung kann im Rahmen der Erfindung frei gewählt werden. Die Anordnung der Auflagerstellen 151, 152 kann auch oberhalb des Niveaus der Beckenkrone 2 erfolgen.

An der Auflagerstelle 151, die durch einen in Richtung zur Beckenmitte wegstehenden Schenkel eines auf der Beckenkrone 2 fixierten Lagerwinkels 150 gebildet ist, ist ein Antriebsmotor 140 des Rührwerks 9 vorgesehen, welcher über ein Antriebselement, hier eine Antriebswelle 190 eine in das Substrat eintauchende Rührschraube 191 antreibt. Die Antriebswelle 190 ist dabei, z.B. durch eine Hohlwelle, gasdicht durch eine Öffnung der Gasmembran 7 hindurchgeführt. Durch die erfindungsgemäße Form der Anbringung der Auflagerstelle 151 können die Auflagerkräfte des Rührwerks 9 in einem ausreichenden Maß aufgenommen werden und zugleich ist die Wartung des Rührwerkes 9 auf einfache Weise möglich.

Auf der gegenüberliegenden Seite des in Fig.1 dargestellten Erdbeckenfermenters 1 ist ein weiteres Rührwerk 9 vorgesehen, dessen Antriebsmotor 140 über einen Lagerblock 193 auf einem auf der Beckenkrone 2 fixierten und von dieser in Richtung zur Mitte des Erdbeckens 19 ragenden Bediensteg 50 angeordnet ist, der eine weitere Auflagerstelle 152 ausbildet, die sich so wie die Auflagerstelle 151 außerhalb der gasdichten Abdeckung befindet. Der Bediensteg 50 ist seinerseits über eine Stütze 181 auf einem Beckenfundamentbereich 180, der auf der Beckensohle ausgebildet ist, abgestützt. Der Bediensteg 50, der einen Teil der gasdichten Abdeckung bildet, ist in dem in das Erdbecken 19 ragenden Teil randseitig mit der Gasmembran 7 gasdicht verbunden.

In dem in Fig.2 gezeigten Ausführungsbeispiel sind an gegenüberliegenden Seiten Führungselemente 35 in Form von Führungsschienen vorgesehen, entlang denen die Rührwerke 9 mit ihren Motoren 140 verschiebbar angeordnet sind, wodurch eine Höhenverstellbarkeit gegenüber der Beckensohle 300 gewährleistet ist. Die Führungselemente 35 sind an ihren unteren Enden auf jeweils einem Beckenfundamentbereich 180 aufgestützt und dort verankert, während sie mit ihren oberen Enden durch die gasdichte Abdeckung 7 hindurchgeführt und erfindungsgemäß im Wesentlichen auf der Höhe der Beckenkrone 2, und zwar auf der einen Seite an der Auflagerstelle 151 und auf der anderen Seite an dem Bediensteg 50, aufgelagert bzw. abgestützt sind. Auf dem Bediensteg 50 ist ein Schacht 17 ausgebildet, der einen Zutritt oder Zugriff durch eine auf dem Steg 50 ausgebildete Durchgangsöffnung (nicht dargestellt) in das Innere des Erdbeckenfermenters 1 ermöglicht, um eine Wartung des entlang des senkrecht verlaufenden Führungselements 35 geführten Rührwerks 9 zuzulassen.

In Fig.3 ist eine weitere Ausführungsform des erfindungsgemäßen Erdbeckenfermenters 1 gezeigt, bei der eine Bedienbrücke 5 das Erdbecken 19 zwischen gegenüberliegenden Beckenkronenbereichen überspannt und dabei eine Auflagerstelle 153 auf der Höhe der Beckenkrone 2 ausbildet, welche das Auflager für die beiden Rührwerke 9 bildet. Die Bedienbrücke 5 ist dabei so angeordnet, dass sie einen Teil der gasdichten Abdeckung des Erdbeckenfermenters 1 bildet und an ihren Rändern teilweise mit der Gasmembran 7 verbunden ist. Durch das Vorsehen der Bedienbrücke 5, die an der Beckenkrone 2 verankert ist, können die beim Betrieb auftretenden Auflagerkräfte der Rührwerke 9 so aufgenommen werden, dass ein zuverlässiger Betrieb auch bei sehr dickflüssigen Substraten durchführbar ist. Das Antriebselement 190 und das Führungselement 35 der Rührwerke 9 sind gasdicht durch eine Öffnung in der Bedienbrücke 5 hindurchgeführt.

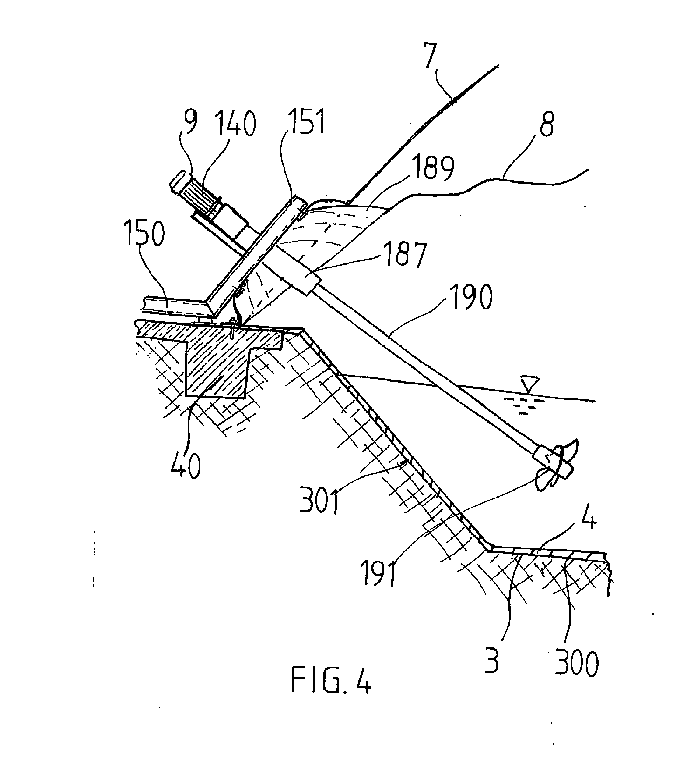

Fig. 4 zeigt die Anwendbarkeit der Erfindung für einen Erdbeckenfermenter mit der einer gasdichten Abdeckung, die durch eine Innen-Gasmembran 8 und eine Außen-Gasmembran 7 zumindest teilweise gebildet ist. Zwischen der Innen-Gasmembran 8 und der Außen-Gasmembran 7 wird über ein Stützgas ein Stützdruck aufrechterhalten, der bei sich verändernder Gasfüllung der Innen-Gasmembran 8 für eine unveränderte Gestalt der Außen-Gasmembran 7 sorgt.

Ein Hohlwellenstück 187 ist gasdicht durch sowohl die Außen-Gasmembran 7 als auch die Innen-Gasmembran 8 hindurchgeführt und ermöglicht in seinem Inneren die Rotation der über den außerhalb der gasdichten Abdeckung angebrachten Antriebsmotor 140 angetriebenen Antriebswelle 190, an deren Ende ein Rührpropeller 191 befestigt ist, der in das Substrat eintaucht. Die Auflagerstelle 151, an der das Hohlwellenstück 187 mit dem Antriebsmotor 140 festgelegt ist und welche über ein Anschlussstück 189 gasdicht mit der Innen- und Außen-Gasmembran 8, 7 verbunden ist, nimmt sämtliche im Betrieb auftretenden Auflagerkräfte auf und leitet sie über den Lagerwinkel 150 an ein Fundament 40, das in der Beckenkrone 2 eingebettet ist, ab.

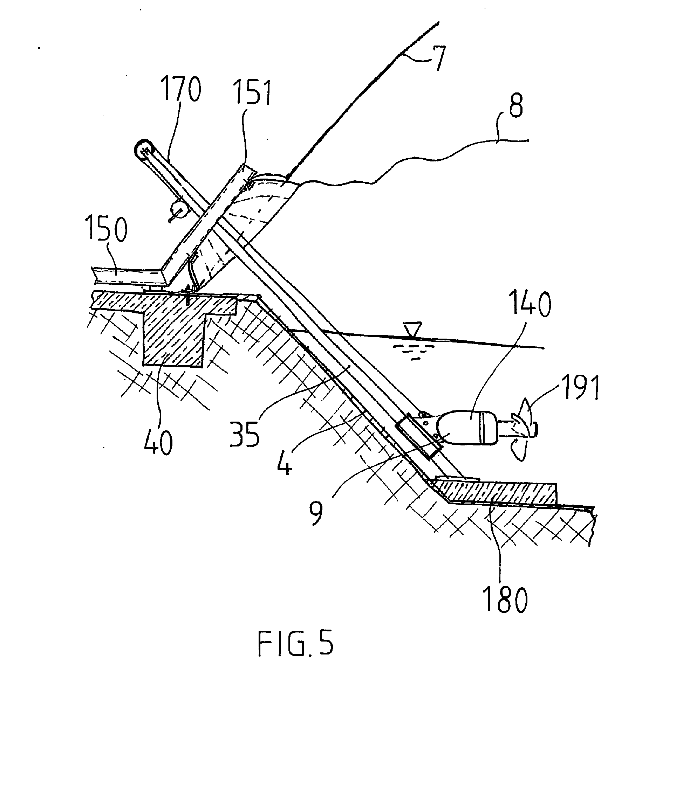

Fig.5 zeigt jene Ausführungsvariante der Erfindung, bei der die auf der Höhe der Beckenkrone oder darüber angeordnete Auflagerstelle 151 das schräg in das Becken verlaufende Führungselement 35 an seinem oberen Ende abstützt und dadurch für eine zuverlässige Höhenverstellbarkeit des Rührwerkes 9 sorgt, wobei das Führungselement 35 durch die Innen- und Außen-Gasmembran 8, 7 gasdicht hindurchgeführt ist. Über ein Seil 170 kann das Rührwerk 9 in verschiedenen Höhen im Becken angeordnet werden und bei Bedarf auch über das Niveau des Substrats gehievt werden.

Fig.6, 7, 8 zeigen eine Ausführungsform des erfindungsgemäßen Erdbeckenfermenters 1, bei der die Zu- und Ableitungen 14, 15, 16 oberhalb des Beckenbodens 3 geführt sind und die obere Auflagerstelle 153 für das Führungs- und Abstützelement 35 der Rührwerke 9 auf der Höhe der Beckenkrone 2 und innerhalb des von dieser umschlossenen Bereiches vorgesehen ist. Dadurch müssen weder die schrägen Seitenwände noch die Beckensohle des Beckenbodens 3 durchstoßen werden. Somit kann die Dichtungsschicht 4 des Beckenbodens 3 durchdringungsfrei ausgeführt sein.

Um ein geeignetes Betreiben und Warten des erfindungsgemäßen Erdbeckenfermenters 1 zu ermöglichen, ist die Bedienbrücke 5 vorgesehen, die das Erdbecken 19 überspannt, wobei im gezeigten Beispiel die Bedienbrücke 5 sich von einer Längsseite des Erdbeckens 19 zur gegenüberliegenden, anderen Längsseite erstreckt und mit ihren Enden auf gegenüberliegenden Bereichen der Beckenkrone 2 aufliegt. Aus statischen Gründen ist die Bedienbrücke 5 dachartig ausgeführt, wie aus Fig.7 ersichtlich ist, sie kann aber in jeder beliebigen anderen Form gestaltet sein. Auf der Bedienbrücke 5 sind mehrere Durchlässe, durch welche die Zu- und Ableitungen 14, 15 und die Gas-Ableitung 16 gasdicht in das darunter liegende Erdbecken 19 hindurchgeführt sind, ausgebildet.

Weiters ist auf der Bedienbrücke 5 zumindest ein dichtend verschließbarer Service-Schacht 17 vorgesehen, der sich von einer Schachtöffnung in der Bedienbrücke 5 nach unten erstreckt und in dem der obere Teil des Führungs- und Abstützelements 35 für die Rührwerke 9 verläuft, welche z.B. als Stab-, Paddel- oder Tauchmotorrührwerk ausgeführt sein können. Die Schachtöffnung kommuniziert mit dem Inneren des Erdbeckens 19. In ihrem Bereich ist die Auflagerstelle 153 für das Führungs- und Abstützelement 35 vorgesehen, welche für die Fixierung desselben auf der Bedienbrücke vorgesehen ist.

Die Bedienbrücke 5 setzt sich im Wesentlichen aus nicht dargestellten Trägern und einer Bodenabdeckplatte 82 zusammen, wobei die Bedienbrücke 5 zusammen mit ihrer Bodenabdeckplatte 82 einen Abschnitt der gasdichten Abdeckung ausbildet. Die Gasmembran 7 besteht im Ausführungsbeispiel gemäß Fig.6, 7 und 8 aus zwei Gasmembranteilen, die an den an die Bodenabdeckplatte 82 angrenzenden Bereichen mit der Bedienbrücke 5 gasdicht verbunden sind, sodass die Bedienbrücke 5 zusammen mit den Gasmembranteilen die gasdichte Abdeckung des Erdbeckens 19 ausbilden. Die gasdichte Verbindung zwischen der Bedienbrücke 5 und den Gasmembranteilen ist durch eine gasdichte Klemmung im Randbereich der Bedienbrücke 5 gebildet.

Fig.9 zeigt eine Form der Randbefestigung der Gasmembran 7, die auf einer auf der Beckenkrone 2 festgelegten Holzlatte 96 gasdicht geklemmt ist, wobei auf dieser Tragegurte 20, welche die Gasmembran 7 stützen, an ihren Enden eingehängt sind. Die Holzlatte 96 ist ihrerseits auf einer Leiste 99 befestigt, die über eine Holzpfahl-Verankerung 30 gegenüber der Beckenkrone 2 festgelegt ist. Weiters ist ein Einbindegraben 55 vorgesehen, um eine feuchtigkeits-isolierende Folie 48 und eine Nagetierschutzabdeckung 49 zu verankern.

Fig.10 zeigt eine Variante dieser Befestigungsvorrichtung, bei der durch eine weitere Latte 97 hindurchgeführte Schrauben 31 für eine sichere Verbindung mit der Beckenkrone 2 sorgen.

In Fig.11 und 12 ist ein weiteres Ausführungsbeispiel des erfindungsgemäßen Erdbeckenfermenters 1 gezeigt, dessen umlaufende Beckenkrone 2 durch ein Stahlbetonfundament 40 gebildet ist, welches einerseits eine Bodenbefestigung und andererseits eine Verankerung für zwei Gasmembranen - eine Innen- und eine Außen-Gasmembran 7, 8 - die Dichtungsschicht 4 und die Feuchtigkeitsisolationsschicht 48 des Erdbeckens 19 bereitstellt. Die Innen- und die Außen-Gasmembran 7, 8 sind an ihren äußeren Rändern mittels eines Winkelprofils 71 gemeinsam gasdicht geklemmt. Die äußere Gasmembran 7 dient dem Schutz gegen äußere Einflüsse und wird durch ein zwischen dieser und der inneren Gasmembran 8 eingeleitetes Stützgas von der inneren Gasmembran 8 beabstandet gehalten. Je nach Gas-Füllstand ist die innere Gasmembran 8 angehoben (strichlierte Linie) oder abgesenkt (ausgezogene Linie). Die am Stahlbetonfundament 40 festgelegten Tragegurte 20 bilden ein Tragnetz und sind vorgesehen, um zu verhindern, dass die innere Membran 8 auf den Boden des Erdbeckens 19 absinkt und dabei verschmutzt oder beschädigt wird.

Das Erdbecken 19 wird beispielsweise hergestellt, indem das Stahlbetonfundament 40 im Erdbereich ausgebildet und dann innerhalb des umlaufenden Stahlbetonfundaments 40 das Erdreich so abgetragen wird, dass die Wandschrägen und die Beckensohle des Beckenbodens 3 ausgebildet werden. Danach wird die Schutzfolie 48 gegen Feuchtigkeit und bedarfsweise eine Wärmedämmungsschicht 47 aufgelegt und darauf die gasdichte Dichtungsschicht 4 aufgebracht, die an ihren Rändern durch eine Klemmvorrichtung 72 auf dem Stahlbetonfundament 40 gasdicht geklemmt ist. Um einen besseren Halt der Dichtungsbahn der Dichtungsschicht 40 zu ermöglichen, ist diese über das Stahlbetonfundament 40 hinaus in einen Einbindegraben 55 geführt, in dem die Ränder der Dichtungsschicht 4 nach unten geführt sind. Der Einbindegraben 55 wird danach verfüllt.

Die Dichtungsschicht 4 wird ohne Durchdringungen verlegt. Somit sind keine Durchführungen oder sonstige Durchlässe vorgesehen, welche die Dichtheit der Dichtungsschicht beeinträchtigen könnten.

Damit kann das innerhalb des Erdbeckenfermenters 1 bei der Fermentation entstehende Gas nicht nach außen entweichen.

Die Bedienbrücke 5' überspannt das Erdbecken 19 von der einen zur gegenüberliegenden Seite der Beckenkrone 2 und ist über zumindest zwei Stützen 10 gegenüber dem Beckenboden 3 abgestützt, der in seinem horizontalen Bereich durch einen Betonfundament-Körper 41 gesichert ist. Auf der begehbar ausgeführten Bedienbrücke 5' sind zwei gasdichte Schächte 17 mit Schachtöffnungen ausgebildet, über welche jeweils das senkrechte Führungselement 35, das an seinem unteren Ende auf dem Betonfundament 41 aufgestützt ist, in die Tiefe führt. Die Auflager 152 für die Fixierung des oberen Teils der Führungselemente 35 sind im Bereich der Schachtöffnungen ausgebildet. Auf diesen Führungselementen 35 sind die Rührwerke 9 höhenverstellbar gehalten. Die Schächte 17 ermöglichen die Steuerung und Wartung der Rührwerke 9 bzw. ihre mechanische Ankopplung. Zusätzlich sind nicht dargestellte Durchlässe in der Bedienbrücke 5' und/oder in den Serviceschächten 17 vorgesehen, durch die Zu- und Ableitungen geführt sind.

Weiters ist in der Bedienbrücke 5' ein nicht dargestelltes Sichtfenster ausgenommen, das gasdicht verschließbar ist.

Im Ausführungsbeispiel gemäß Fig.13 und 14 ist ein Bediensteg 50 vorgesehen, der an einem Ende im Bereich der Beckenkrone 2 befestigt ist und mit seinem freien Ende in das Erdbecken 19 ragt. Der Bediensteg 50 ist durch zwei Träger 89 gebildet, die eine Bodenabdeckplatte 87 tragen und die über Stützen 100 gegenüber dem Betonfundament-Körper 41 des Beckenbodens 3 abgestützt sind. Im Bereich der Beckenkrone 2 liegen die Träger 89 auf Lagern 90 auf.

Auf dem Bediensteg 50 ist ein gasdichter Durchlass 86 ausgebildet, durch welchen die Zu- und Ableitung 14 gasdicht in das darunter liegende Erdbecken 19 hindurchgeführt ist. Weiters ist auf dem Bediensteg 50 ein dichtend verschließbarer Service-Schacht 17 mit einer Schachtöffnung angeordnet, durch welche das Führungselement 35 für das Rührwerk 9 hindurch geführt ist, um an der durch den Steg 50 gebildeten Auflagerstelle 152 festgelegt zu sein.

Im oberen Bereich ist der Service-Schacht 17 mit der Gasableitung 16 verbunden, die mit dem Innenraum des Erdbeckenfermenters 1 kommuniziert. Zur Ausführung von Servicearbeiten ist der Bediensteg 50 begehbar und dafür mit einem Laufrost 81 versehen, der an den Seiten durch ein Schutzgeländer 51 gesichert ist.

Der Bediensteg 50 bildet mit seiner Bodenabdeckplatte 87 einen Abschnitt der gasdichten Abdeckung aus, indem die Bodenabdeckplatte 87 eine Öffnung in der Gasmembran 7 verschließt (Fig.14), wobei die Bodenabdeckplatte 87 mit dem Bediensteg 50 gasdicht verbunden ist, sodass der Bediensteg 50 zusammen mit der Gasmembran 7 die gasdichte Abdeckung des Erdbeckens 19 ausbilden. Anstelle einer einteiligen Gasmembran 7 kann diese auch aus mehreren Teilen zusammengesetzt sein.

Die gasdichte Verbindung zwischen der Bodenabdeckplatte 87 und der Gasmembran 7 ist dabei durch einen entlang des Randbereiches der Bodenabdeckplatte 87 des Bedienstegs 50 verlaufende gasdichte Klemmung 85 gebildet, welche die Berandung der Öffnung in der Gasmembran 7 gasdicht einspannt. Um zu verhindern, dass die Gasmembran 7 mit dem Rührwerk 9 in Kontakt kommt und dabei zerstört wird, sind normal zur Bodenabdeckplatte 87 vorstehende Abstandhalter 60, die aus gebogenen Kunststoffstreifen gebildet sind, entlang des Randes der Bodenabdeckplatte 87 angebracht.

In der Ausführungsform gemäß Fig.15 und 16 ragen die Träger 89 des Bediensteges 50' ohne Stützen in das Erdbecken 19. Für die sichere Verankerung im Bereich der Beckenkrone 2 sorgt ein zusätzliches Widerlager 490, an dem die Träger 89 festgeschraubt sind. Da keine Schächte auf dem Bediensteg 50' ausgebildet sind, genügt eine relativ kleinflächige Bodenabdeckplatte 87', welche anstelle eines Schachtes eine gasdicht verschließbare Bedienöffnung 61 aufweist, über die Sicht bzw. Zutritt in das Erdbecken 19 ermöglicht wird. Im Bereich der Bedienöffnung 61 kann eine Auflagerstelle ausgebildet sein, welche entweder ein Rührwerk lagert oder eine Auflagerstelle für ein Führungselement darstellen kann.

Wie im Ausführungsbeispiel gemäß Fig. 13, 14 verschließt die Bodenabdeckplatte 87' eine Öffnung in der Gasmembran 7 gasdicht und bildet somit zusammen mit der Gasmembran 7 die gasdichte Abdeckung für das Erdbecken 19. Für ausreichende Durchwirbelung des in den gefüllten Erdbeckenfermenter 1 gefüllten Substrats sorgen Gas-Zuleitungen 69 für ein nicht dargestelltes Gasrührwerk. Weiters sind Druckluftzufuhrleitungen 67 über gasdichte Durchlässe durch den Bediensteg 50' hindurch in das Innere des Erdbeckens 19 geführt. Über die Zuleitung 14 wird Substrat, wie z.B. Gülle in den erfindungsgemäßen Erdbeckenfermenter 1 geleitet, oder es kann diese Zuleitung14 für Umpump- oder Spülvorgänge genutzt werden. Über die Ableitung 15 kann das verbrauchte Substrat wieder entnommen werden.

Für die Erhöhung der Fermentationsgeschwindigkeit beispielsweise bei niedrigen Außentemperaturen sind Heizzu- und ableitungen 68 durch den Bediensteg 50' hindurchgeführt, über die dem Erdbecken 19 ein Wärmemedium zuführbar ist.

Fig.17 zeigt eine Ausführungsform der Erfindung, bei der wie in Fig.11, 12 und 13 die Bedienbrücke 5 auf halber Länge das Erdbecken 19 überspannt. Zusätzlich zu den beiden Außenmembranteilen 7 sind entsprechende Innenmembranteile 8 ausgebildet, die zusammen mit den Außenmembranteilen entlang der Beckenkrone 2 und entlang der Längsseiten des Bediensteges 5 gasdicht geklemmt sind, wobei zwischen dem linken Außenmembranteil 7 und dem linken Innenmembranteil 8 genauso wie zwischen dem rechten Außenmembranteil 7 und dem rechten Innenmembranteil 8 eine Stützgas-Atmosphäre für die nach außen gewölbte Form der Außenmembran 7 sorgt. Sowohl im linken als auch im rechten Beckenteil sind Stützträger 101 vorgesehen, welche Stützgurte 20 halten und zusammen mit diesen das vollständige Absinken der Innenmembranteile 8 auf beiden Seiten verhindern. Die Stützträger 101 und die Stütze 10 für die Bedienbrücke 5 sind in Fundamenten 41 gesichert. Auf der Bedienbrücke 5 ist ein Schacht 17 mit einer darin befindlichen Schachtöffnung vorgesehen, in deren Bereich eine Auflagerstelle für ein Rührwerk oder für ein Führungselement desselben vorhanden sein kann.

In Fig.18 und 19 sind verschiedene Formen der gasdichten Klemmung der Außen-und Innenmembran 7, 8 auf der Beckenkrone gezeigt, wobei in Fig.18 die Klemmdichtung mittels Winkelprofils 71 und in Fig.19 diese mit einem U-Profil 77 erfolgt.

## Patentansprüche

1. Erdbeckenfermenter (1) mit einem abgesenkten Beckenboden (3), der mit einer Dichtungsschicht (4) abgedeckt ist und ein Erdbecken (19) ausbildet, wobei zumindest eine gasdichte Abdeckung (7, 8) vorgesehen ist, und wobei Zu- und Ableitungen (14, 15, 16, 67, 68, 69) und zumindest ein Rührwerk (9) vorgesehen sind, **dadurch gekennzeichnet, dass** der Erdbeckenfermenter (1) eine umlaufende Beckenkrone (2) aufweist, die durch ein Fundament gebildet ist, und dass die gasdichte Abdeckung (7, 8) durch eine gasdichte Klemmung auf der Beckenkrone (2) befestigt ist.

2. Erdbeckenfermenter nach Anspruch 1, **dadurch gekennzeichnet, dass** die Klemmdichtung der gasdichten Abdeckung (7, 8) auf der Beckenkrone (2) mittels eines Winkelprofils (71) oder mittels eines U-Profils (77) erfolgt.

3. Erdbeckenfermenter nach Anspruch 1 oder 2, **dadurch gekennzeichnet, dass** die gasdichte Abdeckung zumindest bereichsweise durch zumindest eine Gasmembran (7, 8) ausgebildet ist.

4. Erdbeckenfermenter nach Anspruch 3, **dadurch gekennzeichnet, dass** die Befestigung der zumindest einen Gasmembran (7) an ihrem Rand durch gasdichte Klemmung auf der Beckenkrone (2) erfolgt.

5. Erdbeckenfermenter nach Anspruch 3 oder 4, **dadurch gekennzeichnet, dass** die zumindest eine Gasmembran (7) auf einer auf der Beckenkrone (2) festgelegten Holzlatte (96) gasdicht geklemmt ist, wobei auf dieser Tragegurte (20), welche die Gasmembran (7) stützen, an ihren Enden eingehängt sind.

6. Erdbeckenfermenter nach Anspruch 1, **dadurch gekennzeichnet, dass** die zumindest eine Gasmembran durch eine Außenmembran (8) und eine Innenmembran (7) gebildet ist, wobei die Außenmembran (8) und die Innenmembran (7) in ihren Randbereichen zumindest bereichsweise auf der Beckenkrone (2) gasdicht geklemmt sind.

7. Erdbeckenfermenter nach einem der vorhergehenden Ansprüche 1 bis 6, **dadurch gekennzeichnet, dass** die Beckenkrone (2) durch ein Stahlbetonfundament (40) gebildet ist, welches einerseits eine Bodenbefestigung und andererseits eine Verankerung für die gasdichte Abdeckung (7, 8) bereitstellt.
